# EUROPÄISCHE PATENTSCHRIFT

(11) **EP 1 686 951 B1**
(45) Veröffentlichungstag und Bekanntmachung des Hinweises auf die Patenterteilung: **02.03.2011**
(21) Anmeldenummer: 04818411.3
(22) Anmeldetag: 04.11.2004
(51) Int. Cl.: A61K 31/52, A61K 8/14, A61K 8/42, A61K 8/60, A61K 8/67, A61K 38/58, A61Q 19/00, A61K 36/88, A61P 9/14, A61P 17/00

(54) **KOSMETISCHES ODER THERAPEUTISCHES KOMBINATIONSPRÃPARAT**
COMBINED COSMETIC OR THERAPEUTIC PREPARATION
PREPARATION COMBINEE COSMETIQUE OU THERAPEUTIQUE

(30) Priorität: 11.11.2003 DE 10352602
(43) Veröffentlichungstag der Anmeldung: 09.08.2006
(73) Patentinhaber: ROVI Cosmetics International GmbH, 36381 Schlüchtern (DE)
(72) Erfinder: BLUME, Gabriele, 36396 Steinau a.d. Strasse (DE); TEICHMÜLLER, Dirk, 63589 Linsengericht (DE)
(74) Vertreter: Weber, Roland
(86) Internationale Anmeldenummer: PCT/EP2004/052792
(87) Internationale Veröffentlichungsnummer: WO 2005/046623

(56) Entgegenhaltungen:
- EP-A- 0 366 156
- EP-A- 1 090 629
- WO-A-01/54653
- DE-A1- 4 221 256
- US-A- 5 786 384
- BELCARO G ET AL: "Essaven gel: Review of experimental and clinical data" ANGIOLOGY, Bd. 52, Nr. Supplement 3, Dezember 2001 (2001-12), Seiten S1-S4, XP009043738 ISSN: 0003-3197
- REMACLE J ET AL: "COMPARISON OF DIFFERENT PHLEBOTONICS ON HUMAN ENDOTHELIAL CELLS OF VEINS SUBJECTED TO HYPOXIA" PHLEBOLOGIE, Bd. 44, Nr. 4, 1991, Seiten 881-889, XP009043467 ISSN: 0031-8280

## Beschreibung

Die vorliegende Erfindung betrifft ein kosmetisches oder therapeutisches Kombinationspräparat mit einem Trägersystem aus membranbildenden Lipiden dadurch gekennzeichnet, dass eine Dreifach kombination von Wirkstoffen vorliegt.

Die Gefäßwände des Btutkreisfaufs sind kontinuierlich einer hohen Belastung ausgesetzt. Im arteriellen System werden durch die aktive Pumptätigkeit des Herzens hohe Drücke aufgebaut. Im venösen System kommt es vor allem in den Extremitäten leicht zu einem Rückstau des transportierten Blutes, da der Transport zum großen Teil entgegen der Schwerkraft stattfindet. Im Kapillarsystem (auch als Endstrombahn bezeichnet) wird dem Blutstrom aufgrund des extrem geringen Durchmessers der Kapillaren ein erheblicher Widerstand entgegengesetzt. Die Blutbewegung in der Endstrombahn wird auch als Mikrozirkulation bezeichnet, wobei die Strömungsgeschwindigkeit des Blutes auf ca. 0,5 mm/Sek. abfällt.

Im wesentlichen bestehen Blutgefäße aus drei Schichten. Die innerste Schicht bildet ein einschichtiges Plattenepithel (Endothel) mit aufgelagerter Basalmembran, wobei "einschichtig" bedeutet, daß es sich in der Regel tatsächlich nur um eine einzige Zellschicht handelt. Die mittlere Schicht besteht im wesentlichen aus glatter Muskulatur und elastischen Fasern. Während die Muskulatur für die Verengung bzw. Erweiterung der Gefäße verantwortlich ist, sorgen die Fasern für ihre Elastizität. Die äußerste Schicht ist ebenfalls elastisch und besteht im wesentlichen aus Bindegewebe. Ein Unterscheidungsmerkmal für Arterien und Venen ist, daß die Muskelschicht bei Arterien deutlich dicker ausgebildet ist. Die Muskelschicht der Venen ist dementsprechend dünner, sie kann zum Teil auch ganz fehlen und findet sich vor allem bei den großen Venen.

Die Beeinträchtigung der Stabilität der Gefäßwände führt in der Regel zu einer Abnahme der Gefäßwanddichtigkeit, was eine Reihe von verschiedenen Symptomen zur Folge haben kann. Die diffuse, meist schmerzlose Ansammlung von aus dem Gefäßsystem ausgetretener seröser Flüssigkeit in den Gewebsspalten verschiedener Gewebe wird als Gewebswassersucht bzw. Ödem bezeichnet. Ein eiweißarmes Ödem entsteht zum Beispiel durch eine Erhöhung des intravasalen hydrostatischen Drucks oder durch eine Erniedrigung des intravasalen koltoidosmotischen Drucks. Ein eiweißreiches Ödem ist die Folge der Erhöhung der Gefäßwandpermeabilität. Die Erhöhung der Gefäßwandpermeabilität geht häufig mit einer Dilatation einher und kommt unter anderem im Rahmen von Entzündungen vor. Bei einer lokalen Entzündungsreaktion kommt es zur Transsudation von Plasma und ebenfalls zur Transmigration von Blutzellen.

Ein Hämatom bzw. Bluterguß ist eine traumatisch bedingte Blutansammlung außerhalb der Gefäße (Blutextravasat). Das Hämatom kann sich im Gewebe oder in einem vorgebildeten Hohlraum ausbilden, wo es allmählich gerinnt und teilweise bindegewebsartig durchwachsen wird. Als Hämatom der Körperoberfläche mit den typischen farblichen Veränderungen ist das subkutane Hämatom, ein Bluterguß im Unterhautgewebe, bekannt. Infolge des Hämoglobinabbaus kommt es zu der charakteristischen Verfärbung der anfangs blau-roten Blutflecken zu gelblich-grünlichen Blutbeulen. Neben den Hämatomen, die durch mechanische Beeinträchtigung (vor allem stumpfes Trauma durch Stoßen an harten Gegenständen) entstehen, können sich Blutergüsse auch aufgrund einer Überbelastung des Kapillarsystems infolge von z. B. Streß ausbilden. Ein solches Hämatom kann sich z. B. im unteren Augenlid ausbilden und zu umgangssprachlich als "Dunkle Augenränder" oder "Ringe unter den Augen" bezeichneten dunklen Schatten führen.

Bei der Ausbildung dieser dunklen Schatten muß nicht zwingend Blut aus den Gefäßen austreten. Auch der sehr langsame Blutfluß und die gleichzeitige Abreicherung des Sauerstoffs im Blut, einhergehend mit der für venöses Blut charakteristischen, dunkleren Färbung, kann Ursache für solche Schatten sein. Ähnlich verhält es sich bei den umgangsprachlich als "Besenreiser" oder "Hexenbesen" bezeichneten hellrot bis dunkelviolett gefärbten Äderchen, die zum Beispiel an den Beinen häufig zu beobachten sind.

Die oben beschriebenen Symptome werden häufig unter dem Begriff venöse Insuffizienz zusammengefaßt. Venöse Insuffizienz geht oft mit Schmerzen, Spannungs- und Schweregefühl einher. In den Beinen können auch Schwellungen ausgeprägt sein, besonders abends und an heißen Tagen. Duch die Wassereinlagerung kommt es ferner zu einer Minderversorgung des Gewebes mit Sauerstoff. Im folgenden wird der Symptomenkomplex, der aufgrund einer erhöhten Gefäßwandpermeabilität mit der Ausbildung von Hämatomen und Ödemen im Gewebe einhergeht, als Gefäßwandinsuffizienz bezeichnet und umfaßt damit sowohl das venöse als auch das arterielle System.

Auch wenn wissenschaftlich noch nicht ganz geklärt ist, warum Gefäßwände, wie zum Beispiel im unteren Augenlid, schwach werden, sind für die prophylaktische bzw. therapeutische Behandlung von Symptomen, die durch Störungen der Gefäßwandstabilität bedingt sind, aus dem Stand der Technik einige Wirkstoffe bekannt. Zu diesen Wirkstoffen zählen unter anderem Naturstoffe aus den Gruppen der Polyphenole (z.B. Flavonoide) und Triterpene (Saponine). Diese werden als Reinsubstanzen, Stoffgemische bzw. Pflanzenextrakte in Form von Tees, Tabletten, Cremes oder Gelen verabreicht.

Zu den wirksamen Flavonoiden zählt das Rutin, eine Verbindung, die unter anderem aus dem Buchweizen bekannt ist. Neben dem Rutin (auch als Rutosid bezeichnet) werden auch Derivate wie z. B. das Troxerutin (Trihydroxyethylrutin) verwendet sowie weitere partialsynthetisch gewonnene Hydroxyglycoside. Das Flavonoid Rutin findet sich unter anderem im Kraut des Buchweizens (*Fagopyrum esculentum).* Weitere Wirkstoffe, die in die Gruppe der Flavonoide fallen, sind die Anthocyan- und Flavonoidgemische aus Präparaten, die definierte Extrakte des roten Weinlaubs enthalten, und das Diosmin, das unter anderem aus den Schalen von Zitrusfrüchten gewonnen wird.

Zu den vasoprotektiv wirkenden Saponinen zählen die Ruscogenine aus dem Mäusedorn *(Ruscus aculeafus)* und das Saponingemisch Aescin aus den Samen der Roßkastanie *(Aesculum hippocastanum).*

Das Prinzip der aus dem Stand der Technik bekannten Präparate zur Behandlung von Gefäßwandinsuffizienz besteht vor allem darin, mit den oben genannten Wirkstoffen lediglich die Stabilität der Gefäßwände zu erhöhen und so ihre Permeabilität für feste und flüssige Blutbestandteile zu verringern. So beruht die membranstabilisierende Wirkung der Flavonoide vom Rutin-Typ vermutlich auf der Hemmung der Hyaluronidase. Die Hyaluronsäure ist einer der membranstabilisierenden Bestandteile der Bindegewebsschicht. Die Hyaluronidase ist ein körpereigenes Enzym, das den Abbau der Hyaluronsäure im Bindegewebe katalysiert, und die Hemmung dieses Enzyms führt zu einer Verschiebung des enzymatischen Gleichgewichts mit der Folge, daß die körpereigenen Prozesse, die die Membranstabilität erhöhen, das Gleichgewicht dominieren. Die Ruscogenine wirken tonisierend auf die Venen, während bei den Arterien eher die Dilatation gefördert wird. Außerdem hemmen Ruscogenine *in vitro* deutlich das Enzym Elastase. Die Elastase wird für die hydrolytische Spaltung der extrazellulären Matrix und der Endothelzellmembranen an den Gefäßen verantwortlich gemacht. Das Saponingemisch Aescin hemmt ebenfalls die Elastase und zusätzlich die Collagenase, die den Abbau der Bindegewebsgrundsubstanz Collagen katalysiert. Aescin weist signifikante vasoprotektive (Stärkung schwacher Venen) und venentonisierende Effekte (Vorbeugung von Gefäßundichtigkeiten) auf. In klinischen Studien mit Patienten, die an chronischer venöser Insuffizienz (CVI) leiden, wurde gezeigt, daß Aescin die Stabilität der Kapillaren verbessert.

Ein Problem ist, daß die oben genannten Wirkstoffe in ihrer ursprünglichen Form sehr polar sind, und bei den genannten Applikationsformen ist es zweifelhaft, ob der Wirkstoff in allen Fällen tatsächlich an den gewünschten Wirkort gelangt, um dort seine Wirkung zu entfalten. Ein weiteres Problem der derzeit verfügbaren Präparate ist, daß der Symptomenkomplex, der Gefäßwandinsuffizienz, der unter anderem mit Hämatomen bzw. Ödemen einhergeht, in der Regel nur mit Wirkstoffen behandelt wird, die vasoprotektive bzw. venentonisierende Eigenschaften haben.

Es besteht daher der Bedarf für ein Präparat, dessen Wirkungen möglichst den ganzen Symptomenkomplex, der mit Gefäßwandinsuffizienz einhergeht, abdeckt. Hierbei soll das Präparat gewährleisten, daß die Wirkstoffe tatsächlich den gewünschten Wirkort erreichen, um dort ihre Wirkung zu entfalten.

Diese Aufgabe wird gemäß der vorliegenden Erfindung gelöst durch ein kosmetisches oder therapeutisches Kombinationspräparat mit einem Trägersystem aus membranbildenden Lipiden und einer Dreifach kombination von Wirkstoffen, wobei die Wirkstoffe aus den Gruppen Antikoagulantien und mikrozirkuladonsfördemde Stoffe und Vasoprotektiva enthalten sind, wobei der wenigstens eine mikrozirkuladonsfördernde Stoff unter Koffein, Naftidrofuryl, Pentoxifyllin, Buflomedil, Ginkgowirkstoffen und Gemischen davon ausgewählt ist und wobei die Vasoprotektiva unter Aescin, Rutin, Diosmin, Ruscogenin und Gemischen davon ausgewählt sind.

Antikoagulantien sind Stoffe, die die Blutgerinnung hemmen. Um z. B. die Auflösung eines Blutgerinnsels oder eines Hämatoms zu beschleunigen, ist der Einsatz von Antikoaguilantien vorteilhaft. Die Funktion der Vasoprotektiva ist vor allem eine prophylaktische und bewirkt eine Stabilisierung der Gefäßwand, was eine Verbesserung der Gefäßwanddichte und eine Verringerung der Permeabilität für Blutbestandteile zur Folge hat Mikrozirkulationsfördernde Stoffe stimulieren die Durchblutung im Kapillarbereich der sogenannten Endstrombahn. Diese Durchblutungsförderung ist insbesondere vorteilhaft für die Prozesse, die beim Abbau von Hämatomen und Ödemen auftreten. Die, Kombination der genannten Wlrkstoffgruppen untereinander gemäß der vorliegenden Erfindung liefert eine Vielzahl von vorteilhaften Wirkstoffkombinationen für die kosmetische bzw. prophylaktische oder therapeutische Anwendung bei Symptomenkomplexen, die mit der Ausbildung von Ödemen oder Hämatomen einhergehen, wie z.B. der Gefäßwandinsuffizienz.

Gemäß der vorliegende Erfindung werden diese Wirkstoffe mit einem Trägersystem aus membranbildenden Lipiden kombiniert. Dieses Trägersystem dient im wesentlichen als Transportsystem für die genannten Wirkstoffkombinationen. Dieses erfindungsgemäße Transportsystem gewährleistet, daß die Wirkstoffe bei ihrer Anwendung tatsächlich an den gewünschten Wirkort gelangen, um dort ihre Wirkung zu entfalten.

Die Wirkstoffkombinationen gemäß der Erfindung zeichnet sich dadurch aus, daß Wirkstoffe aus den Gruppen Antikoagulantien (a) und Vasoprotektiva (b) enthalten sind. Die Kombination von Vasoprotektiva und Antikoagulantien ist von daher vorteilhaft, da durch die Vasoprotektiva prophylaktisch und gegebenenfalls auch therapeutisch die Stabilität der Gefäßwände erhöht wird, während die Antikoagulantien lokal die Ausbildung von Hämatomen und Gerinnseln verhindern und den Abbau von gegebenenfalls bereits vorhandenen Gerinnseln bzw. Hämatomen fördern.

Da die Wirkstoffe gemäß der Erfindung aus den Gruppen Antikoagulantien (a) und mikrozirkulationsfördernde Stoffe (c) enthalten sind, wird die blutgerinnungshemmende Wirkung der Antikoagulantien vorteilhaft durch die durchblutungsfördernde Wirkung der mikrozirkulationsfördernden Stoffe gefördert.

Bei der vorliegenden Erfindung sind Wirkstoffe aus den Gruppen Vasoprotektiva (b) und mikrozirkulationsfördernde Stoffe (c) enthalten. Diese Kombination ermöglicht es, die Mikrozirkulation zu steigern, und gleichzeitig durch die gefäßwandstabilisierende Wir kung der Vasoprotektiva zu gewährleisten, das die Durchblutungsförderung nicht mit einer gesteigerten Transsudation einhergeht.

Die vorliegende Erfindung ist dadurch gekennzeichnet, daß die Wirkstoffe aus den Gruppen Antikoagulantien (a), Vasoprotektiva (b) und mikrozirkulationsfördernde Stoffe (c) ausgewählt sind. Die Dreifachkombination dieser Wirkstoffe stellt eine optimale Wirkstoffkombination für die kosmetische bzw. prophylaktische oder therapeutische Behandlung von Symptomenkomplexen dar, die mit der Ausbildung von Ödemen oder Hämatomen einhergehen, wie z.B. der Gefäßwandinsuffizienz.

Das Trägersystem des Kombinationspräparates ist vorzugsweise vesikulär. Unter einem esikutären Trägersystem aus membranbildenden Lipiden werden im Sinne der vorliegenden Erfindung Doppelschichtmembranvesikel bzw. einschichtige Nanopartikel verstanden. Zu den Doppel- bzw. auch mehrschichtigen Vesikeln zählen auch die sogenannten Liposome. Hierbei können die Wirkstoffe sowohl im Innenraum der Vesikel in einer Lösung vorliegen, als auch in oder zwischen den Schichten eingelagert vorliegen. Darüber hinaus kann das Trägersystem auch im Sinne der Erfindung im nichtvesikulären Zustand, z. B. als Aggregat von mehreren Schichten, als Trägersystem für die Wirkstoffe fungieren.

Die membranbildenden Lipide des Trägersystems des erfindungsgemäßen Kombinationspräparats umfassen vorzugsweise die membranbildenden Lipide aus den Gruppen der Phospholipide, Ceramide und Diacylglykoside. Wenn es zweckmäßig erscheint, können membranbildende Lipide aus verschiedenen Gruppen in Form von Gemischen miteinander kombiniert werden.

Bei der Verwendung von Gemischen verschiedener Stoffe aus den Gruppen der membranbildenden Lipide ist es bevorzugt, wenn die membranbildenden Lipide wenigstens 70 Gew.-% Phosphatidylcholin enthalten. Besonders bevorzugt ist es, wenn die membranbildenden Lipide etwa 80 bis 90 Gew.-% Phosphatidylcholin enthalten. Der Anteil an Phosphatidylcholin an den membranbildenden Lipiden beeinflußt entscheidend die Transporteigenschaften und die Stabilität des Trägersystems. Phosphatidylcholingehalte von unter etwa 70 Gew.-% liefern ein Trägersystem, das in vesikulärer Form unzureichende Vesikelstabilität aufweist. Je nach zu transportierendem Wirkstoff oder transportierenden Wirkstoffen und je nach erforderlichem Wirkort, an dem die Wirkstoffe freigesetzt werden sollen, um dort ihre Wirkung zu entfalten, kann der Gew.-%-Anteil an Phosphatidylcholin variiert werden. Bei einem Phosphatidylcholinanteil von etwa 80 Gew.-% penetriert das Trägersystem mit den Wirkstoffen die Haut und setzt dort mit Tiefenwirkung sowohl hydrophile als auch lipophile Wirkstoffe frei. Bei einer Erhöhung des Phosphatidylcholingehalts im Trägersystem über 80% nimmt die Tiefenwirkung schrittweise ab. Dies kann gewünscht sein, für den Fall, daß eine ausgesprochen tiefe Wirkung der Wirkstoffe nicht erforderlich ist, sondern daß die Wirkstoffe ihre Wirkung eher in den weiter oben liegenden Schichten entfalten sollen.

Die für das erfindungsgemäße Kombinationspräparat in Betracht kommenden Antikoagulantien umfassen Heparine, Fucoidane, Hirudine, Cumarine und Gemische davon. Man unterscheidet zwischen direkten Antikoagulantien, die unmittelbar mit den Gerinnungsfaktoren wechselwirken, und indirekten Antikoagulantien, die die Synthese von Gerinnungsfaktoren unterbinden. Alle diese Substanzen verhindern die Bildung von Blutgerinnseln und erleichtern damit die Durchblutung v.a. im Kapillarbereich. Für die dermale Applikation werden vor allem die direkt agierenden Makromoleküle wie Heparine, Fucoidane und Hirudine sowie synthetisch hergestellte niedermolekulare Pentapeptide eingesetzt. Ein Beispiel für ein indirektes Antikoagulans ist die Acetyisalicylsäure.

Unter Heparinen werden gemäß der vorliegenden Erfindung sowohl hochmolekulare als auch niedermolekulare Heparine verstanden sowie vergleichbar wirkende Verbindungen, die z. B. Antithrombin III oder den Blutgerinnungsfaktor Xa hemmen. Die erfindungsgemäßen Fucoidane umfassen ebenfalls die hochmolekularen und niedermolekularen Fucoidane. Unter Hirudin werden gemäß der vorliegenden Erfindung Hirudine aus Blutegelextrakten, sowie die Rohextrakte bzw. auch gereinigte Extrakte von Blutegeln, kleinere Hirudine und gentechnologisch hergestellte, rekombinante (r-)Hirudine sowie andere Substanzen, die das aktive Zentrum von Thrombin blockieren, verstanden. Die Bezeichnung Cumarin umfaßt gemäß der Erfindung Antikoagulantien vom Cumarintyp, vom Cumarin abgeleitete Hemmstoffe der Blutgerinnung und andere Wirkstoffe, deren Wirkung auf der Strukturähnlichkeit mit dem Vitamin K (kompetitive Hemmung) basieren.

Bei einer bevorzugten Ausführungsform der vorliegenden Erfindung ist als Antikoagulans Fucoidan enthalten. Besonders bevorzugt ist ein Fucoidangehalt in einer Menge von 0,1 bis 10 Gew.%. Unterhalb von etwa 0,1 Gew.-% wird keine zufriedenstellende Wirksamkeit festgestellt, während oberhalb von 10 Gew.-% die Löslichkeit der begrenzende Faktor ist.

Bei einer weiteren bevorzugten Ausführungsform ist als Antikoagulans niedermolekulares Fucoidan (LMD) enthalten. Besonders bevorzugt ist hierbei eine Menge von 0,1 bis 10 Gew.-%.

Ein erfindungsgemäßes Kombinationspräparat enthält Vasoprotektiva, die Aescin, Rutin, Diosmin, Ruscogenin und Gemische davon umfassen. Unter Aescin werden gemäß der vorliegenden Erfindung Saponine und Saponingemische vom Aescintyp verstanden. Zusätzlich umfaßt der Begriff auch Roßkastaniensamentrockenextrakte, die auf Aescin standardisiert sind. Mit Rutin werden sowohl Rutin selbst als auch weitere Rutoside, Oxyrutine, wie z. B. Troxerutin, sowie weitere Hydroxyethylrutoside und partialsynthetisch gewonnene Hydroxyglycoside des Rutins verstanden. Die Bezeichnung Ruscogenin umfaßt Stoffe aus der Gruppe der Ruscogenin-Saponine sowie auf Ruscogenine standardisierte Extrakte des Mäusedorns. Darüber hinaus kommen als Vasoprotektiva auch definierte Extrakte des roten Weinlaubs in Betracht.

Bei einer bevorzugten Ausführungsform enthält das erfindungsgemäße Kombinationspräparat als Vasoprotektivum Aescin. Besonders bevorzugt ist ein Aesingehalt von 0,1-7 Gew.-%. Im Bereich von weniger als 0,1 Gew.-% ist kein hinreichender vasoprotektiver Effekt feststellbar und oberhalb von 7 Gew.-% treten Löslichkeitsprobleme auf.

Bei der vorliegenden Erfindung umfassen die mikrozirkutationsfördernden Stoffe Koffein, Naftidrofuryl, Pentoxifyllin, Buflomedil sowie Ginkgowirkstoffe und Gemische davon. Unter Ginkgowirkstoffen werden in diesem Zusammenhang standardisierte Extrakte von Ginkgo sowie daraus gewonnene mikrozirkulationsfördernde Fraktionen oder Reinsubstanzen verstanden.

Bei einer bevorzugten Ausführungsform des erfindungsgemäßen Kombinationspräparates ist als mikrozirkulaßonsfördernder Stoff Koffein enthalten. Besonders bevorzugt ist hierbei ein Gehalt von 0,1 bis 2 Gew.-%. Unterhalb von etwa 0,1 Gew.-% tritt keine vorteilhafte Wirkung auf, während oberhalb von 2 Gew.-% Löslichkeitsprobleme auftauchen.

Eine weitere besonders bevorzugte Ausführungsform des erfindungsgemäßen Kombinationspräparates enthält Aescin, vorzugsweise in einer Menge von 4,0 bis 6,0 Gew.-%, besonders bevorzugt 5,0 Gew.-%, niedermolekulares Fucoidan, vorzugsweise in einer Menge von 1,0 bis 3,0 Gel.-%, besonders bevorzugt 2,0 Gew.-%, und Koffein, vorzugsweise in einer Menge von 0,5 bis 1,5 Gew.-%, besonders bevorzugt 1,0 Gew.-%. Die Kombination dieser drei Wirkstoffe aus insgesamt drei verschiedenen Wirkstoffgruppen liefert in den angegebenen Gehalten im Zusammenhang mit dem erfindungsgemäßen Trägersystem ein Kombinationspräparat, das für die kosmetische bzw. prophylaktische oder therapeutische Anwendung zur Behandlung von Symptomenkomplexen optimiert ist, die mit der Ausbildung von Ödemen oder Hämatomen einhergehen, wie z.B. der Gefäßwandinsuffizienz.

Eine bevorzugte Ausführungsform des Kombinationspräparates gemäß der Erfindung ist dadurch gekennzeichnet, daß das Trägersystem Linoisäure in stabilisierter Form, vorzugsweise in einem Gehalt von 2,5 bis 4,5 Gew.-% enthält. Unter Linolsäure in stabilisierter Form wird in diesem Zusammenhang verstanden, daß die Linolsäure als Bestandteil des Trägersystems in dem Trägersystem stabilisiert ist. Das heißt, stabilisierte Linolsäure liegt hier in Form des Fettsäurebestandteils Linolsäure der Membranlipide gebunden vor. Dadurch wird verhindert, daß die Linolsäure durch körpereigene Prozesse chemisch modifiziert wird und so ihre Wirkung verliert. Linolsäure ist eine der unter der Bezeichnung Vitamin F zusammengefaßten, essentiellen Fettsäuren. Diese sind unter anderem Bestandteil der Membranbausteine der menschlichen Haut, und die Zuführung von zusätzlicher Linolsäure verlangsamt die Alterungsprozesse (z. B. Faltenbildung) der menschlichen Haut.

In einer weiteren bevorzugten Ausführungsform enthält das erfindungsgemäße Kombinationspräparat neben den genannten Wirkstoffen weiterhin wenigstens einen Thermorezeptor-Agonisten, der aus der Gruppe ausgewählt ist, die natürliches oder synthetisches Capsaicin, bevorzugt in einer Menge von 0,1 bis 1 Gew.-%, besonders bevorzugt in einer Menge von 0,2 bis 0,6 Gew.-% und Nicotinsäure, Nicotinsäureamid, Nicotinsäureester oder Gemische davon, bevorzugt in einer Menge von 0,5 bis 5 Gew.-%, besonders bevorzugt in einer Menge von 0,5 bis 3 Gew.-%, umfaßt. Die Funktion des Thermorezeptor-Agonisten im Kombinationspräparat ist es, bei der Anwendung über die Anregung der Thermorezeptoren einen durchblutungsfördernden Effekt für die behandelte Körperstelle zu erzielen. Zusätzlich zur Wirkung eines enthaltenen mikrozirkulationsfördernden Mittels wird hierdurch auch die Durchblutung in größeren Blutgefäßen gefördert. Für die Verwendung eines erfindungsgemäßen Kombinationspräparates am Auge ist aufgrund der großen Empfindlichkeit des Auges gegebenenfalls von der Zugabe eines Thermorezeptor-Agonisten abzusehen. Das erfindungsgemäße Kombinationspräparat mit Thermorezeptor-Agonisten eignet sich jedoch beispielsweise mit Vorteil zur Behandlung von Gefäßinsuffizienz in Raucherbeinen.

Um das erfindungsgemäße Kombinationspräparat zu konservieren, enthält eine weiterhin bevorzugte Ausführungsform der vorliegenden Erfindung 10- 25 Gew.-% Ethanol.

Zur Herstellung eines Kombinationspräparates gemäß der vorliegenden Erfindung werden zunächst die wasserlöslichen Wirkstoffe aus den oben genannten Gruppen der Antikoagulantien, Vasoprotektiva, mikrozirkulationsfördernden Stoffe und/oder Thermorezeptor-Agonisten in entsprechenden Mengen unter Rühren in Wasser bei maximal 40°C klar gelöst. In einem weiteren Vorbereitungsschritt werden die fettlöslichen Wirkstoffe aus den oben genannten Gruppen der Antikoagulantien, Vasoprotektiva, mikrozirkulationsfördernden Stoffe und/oder Thermorezeptor-Agonisten in entsprechenden Mengen unter Rühren in einer ethanolischen Lecithinlösung bei maximal 50°C klar gelöst. Die beiden vorbereiteten Lösungen werden langsam unter Turraxieren (= Homogenisieren unter Verwendung eines Turrax-Homogenisators) zusammengeführt und anschließend durch Hochdruckhomogenisation, Extrusion und/oder anderweitige mechanische Zerkleinerung auf eine Vesikeldurchmessergröße von maximal 500 nm gebracht. Unter stetigem Homogenisieren wird dann wässriger Phosphatpuffer zugesetzt und solange weiter homogenisiert, bis eine leichtviskose, homogene Emulsion entsteht. Der pH-Wert der Emulsion wird erforderlichenfalls mit herkömmlichen Mitteln auf etwa pH 6,5 bis 7,5 eingestellt.

Ein erfindungsgemäßes Kombinationspräparat wird vorzugsweise in eine kosmetische oder pharmazeutische Trägermatrix eingearbeitet, besonders bevorzugt in einer Einsatzkonzentration von 1,0 bis 5,0 Gew.-%. Bei der Trägermatrix kann es sich um Gelformulierungen, Cremeformulierungen (O/W- und W/O-Emulsionen), Lotionen, Maskenanwendungen etc. handeln.

Ein Verfahren zur Formulierung eines Kombinationspräparates gemäß der vorliegenden Erfindung in Form eines Gels ist in folgender Weise zu beschreiben: unter leichtem Rühren werden ein Verdikker, vorzugsweise in einer Menge von 0,1 bis 3,0 Gew.-%, und ein nichtionischer Emulgator, vorzugsweise in einer Menge von 1,0 bis 15,0 Gew.-%, und bei einer besonders bevorzugten Ausführungsform ein Co-Emulgator in Wasser vollständig gelöst. In diese Matrix wird bei maximal 30°C eine der oben beschriebenen Ausführungsformen des erfindungsgemäßen Kombinationspräparates, vorzugsweise in einer Menge von 1,0 bis 5,0 Gew.-%, homogen eingerührt. Abschließend wird ein Konservierungsmittel, vorzugsweise in einer Menge von 0,1 bis 0,5 Gew.-%, zugesetzt und im weiteren homogen eingerührt. Das Gel zeigt klare bis trübe Erscheinungsform. Die Viskosität variiert in Abhängigkeit von Art und Einsatzkonzentration des verwendeten Verdickers. Der pH-Wert des Gels wird erforderlichenfalls mit herkömmlichen Mitteln auf etwa 5,5 bis 6,5 eingestellt.

Vorzugsweise werden membranbildende Lipide und eine Dreifachkombination von Wirkstoffen, die aus den Gruppen (a) Antikoagulantien, (b) Vasoprotektiva und (c) mikrozirkulationsfördernde Stoffe ausgewählt sind, sowie in einer besonders bevorzugten Ausführungsform weiterhin wenigstens ein Thermorezeptor-Agonist, zur Herstellung eines Kosmetikums oder Arzneimittels für die Prophylaxe und/oder die Behandlung von Hämatomen, vorzugsweise von Hämatomen des unteren Augenlids, und/oder von Venenteiden verwendet.

Weitere Vorteile, Merkmale und Anwendungsmöglichkeiten der vorliegenden Erfindung werden aus den folgenden Beispielen und der dazugehörigen Figur deutlich.

### Beispiel 1

Ein erfindungsgemäßes Kombinationspräparat wird nach dem oben genannten Verfahren hergestellt und umfaßt folgende Bestandteile:

| | |
|---|---|
| 16,0 Gew.-% | Ethanol, unvergällt (Bundesmonopolverwaltung für Branntwein, DE) |
| 10,0 Gew.-% | Phospholipide (Lecithin/PL 80) |
| 5,0 Gew.-% | Aescin (Synopharm GmbH, D-22885 Barsbüttel) |
| 2,0 Gew.-% | Fucoidan (Algenextrakt hochrein, Kraeber GmbH & Co, D-25474 Ellerbek) |
| 1,0 Gew.-% | Koffein |
| 0,5 Gew-% | Kaliumdihydrogenphosphat |
| ad 100 Gew.-% | Wasser |

Zunächst wurden Fucoidan und Koffein bei 40°C unter Erhalt einer klaren, schwach gelblichen Lösung vollständig in Wasser gelöst. Gleichzeitig wurde in einer klaren, braunen ethanolischen Lecithinlösung das Aescin bei einer Temperatur von maximal 50°C vollständig aufgelöst. Der Puffer wurde hergestellt, indem Kaliumdihydrogenphosphat unter Rühren in Wasser vollständig gelöst wurde. Der pH-Wert dieser Lösung wurde mit NaOH-Lösung auf 11,0 bis 12,0 eingestellt. Unter Turraxieren (= Homogenisieren unter Verwendung eines Turrax-Homogenisators bei 10.000 U/min) wurde nun die ethanolische Lecithin-/Aescin-Lösung langsam der wässrigen Fucoidan-/Koffein-Lösung zugefügt und im Anschluß durch ein 200 nm Polycarbonatfilter extrudiert. Unter stetigem Homogenisieren wurde abschließend der Phosphatpuffer zugesetzt und solange weiter homogenisiert, bis eine beige, leichtviskose, homogene Emulsion entstand. Der pH-Wert der Emulsion betrug 6,7. Die Vesikelgröße, ausgedrückt als Durchmesser der Liposomen-Hohlkugeln, wurde mit einem Zetarnaster S der Fa. Malvern Instruments, UK nach dem Verfahren der Photonen-Korrelations-Spek -oskopie (PCS) mit 152 nm bestimmt. Wurde der angestrebte pH-Wert nicht unmittelbar erreicht, so konnte erforderlichenfalls mit NaOH-Lösung auf einen pH-Wert von 6,5 bis 7,5 eingestellt werden.

### Beispiel 2

Das erfindungsgemäße Kombinationspräparat nach Beispiel 1 wurde in einer Einsatzkonzentration von 5,0 Gew.-% in eine Gelformulierung eingearbeitet. Erfindungsgemäß geeignet sind beispielsweise Einsatzkonzentration von 1,0 bis 5,0 Gew.-%. Das Gel ist nur ein Beispiel für eine erfindungsgemäß geeignete kosmetische oder pharmazeutische Trägermatrix.

Die Formulierung nach Beispiel 2 umfaßt folgende Bestandteile:

| | |
|---|---|
| 1,5 Gew.-% | Verdicker (Acritamer^{®}; Fa. R.I.T.A.,USA) |
| 4,4 Gew.-% | NaOH-Lösung 10% |
| 5,0 Gew.-% | Emulgator (Ritabate^{®}; Fa. R.I.T.A. ,USA) |
| 5,0 Gew.-% | erfindungsgemäßes Kombinationspräparat nach Beispiel 1 |
| 0,2 Gew.-% | Konservierungsmittel (Euxyl K 400^{®}, Fa. Schülke & Mayr, DE) |
| ad 100 Gew.-% | Wasser |

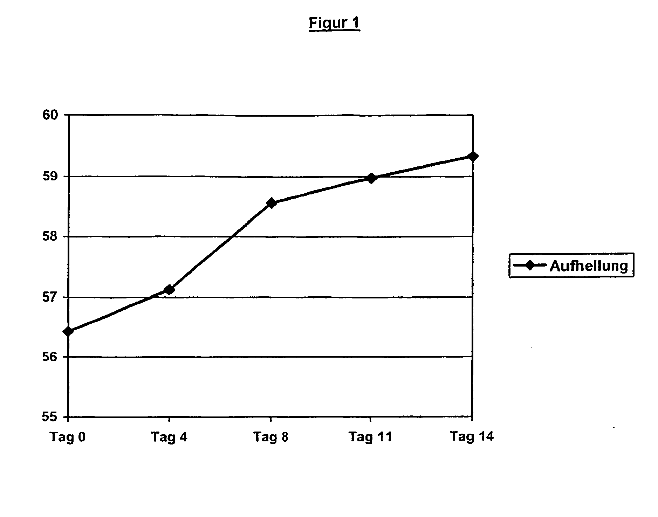

Zunächst wurde der Verdicker unter Rühren bei Raumtemperatur vollständig in Wasser zu einem trüben, hochviskosen Gel gelöst. Im Anschluß wurde der pH-Wert dieses Gels mit 10%iger NaOH-Lösung von etwa 3,3 auf 5,8 angehoben. Daraus resultierte ein klares, schnittfestes Gel. Nacheinander wurden nun der Emulgator, das erfindungsgemäße Kombinationspräparat und das Konservierungsmittel bei maximal 30°C in die Gelmatrix eingerührt und weitere 20 Minuten nachgerührt. Das erhaltene trübe, leicht gelbliche Gel war von schnittfester Konsistenz und hatte einen pH-Wert von 5,8. Wurde der angestrebte pH-Wert nicht unmittelbar erreicht, so konnte er erforderlichenfalls durch Zugabe von NaOH-Lösung auf einen Wert von 5,6 bis 6,0 eingestellt werden. Das Ergebnis der Anwendung einer Zubereitung des erfindungsgemäßen Kombinationspräparates nach Beispiel 2 ist in Figur 1 wiedergegeben.
- Figur 1: zeigt das Ergebnis der Anwendung einer Zubereitung des erfindungsgemäßen Kom- binationspräparates nach Beispiel 2.

Ein gemäß Beispiel 2 hergestelltes Kombinationspräparat wurde 8 Probanden mit Hämatomen im unteren Augenlid ("Ringe unter den Augen") 1 mal täglich in einer Menge von jeweils 0,1 Gramm aufgetragen. Die Färbung der behandelten Hautpartie wurde vor und während der Behandlung mit einem Chromameter CR-300 (Fa. Minolta, Japan) gemessen. In Figur 1 sind die Durchschnittswerte der Färbungen aller Probanden gegen die Behandlungszeit in Tagen dargestellt. Der L*-Wert repräsentiert im Farbraum nach dem L*a*b*-Farbsystem die z-Koordinate und spiegelt den Helligkeitswert der zu messenden Oberfläche wider. Schwarz ist dabei ei einem L*-Wert von 0 und Weiß einem L*-Wert von 100 zuzuordnen. Aus Figur 1 wird der aufhellende Effekt infolge der positiven Wirkung des erfindungsgemäßen Kombinationspräparates auf ein Hämatom des unteren Augenlids deutlich. Über einen Behandlungszeitraum von 14 Tagen ist eine erhebliche Aufhellung des unteren Augenlids feststellbar, wobei die Aufhellung innerhalb von 8 Tagen ab Behandlungsbeginn am stärksten ist und anschließend langsamer voranschreitet.

## Patentansprüche

1. Kosmetisches oder therapeutisches Komblnatlonspräparat mit einem Trägersystem aus membranbildenden Lipiden und Wirkstoffen, **dadurch gekennzeichnet, daß** eine Dreifachkombination von Wirkstoffen vorliegt, die Wirkstoffe aus den Gruppen Antikoagutantien und mikrozirkulationsfördernde Stoffe und Vasoprotektiva enthält, wobei der wenigstens eine mikrozirkulationsfördernde Stoff unter Koffein, Naftidrofuryl, Pentoxifyllin, Buflomedil, Ginkgowirkstoffen und Gemischen davon ausgewählt ist und das wenigstens eine Vasaprotektivum unter Aescin, Rutin, Diosmin, Ruscogenin und Gemischen davon ausgewählt ist.

2. Kombinationspräparat nach Anspruch 1, **dadurch gekennzeichnet, daß** das Trägersystem veslkulär ist.

3. Kombinationspräparat nach einem der Ansprüche 1 oder 2, **dadurch gekennzeichnet, daß** die membranbildenden Lipide die Gruppen der Phospholipide, Ceramide und Diacylglykoside und Gemische davon umfassen.

4. Kombinationspräparat nach einem der Ansprüche 1 bis 3, **dadurch gekennzeichnet, daß** die membranbildenden Lipide wenigstens 70 Gew.-% Phosphatidylcholin, vorzugsweise etwa 80 - 90 Gew.-% Phosphatidylcholin enthalten.

5. Kombinationspräparat nach einem der Ansprüche 1 bis 4, **dadurch gekennzeichnet, daß** die Antikoagulantien unter Heparinen, Fucoidanen, Hirudinen, Pentapeptiden, Cumarin-Derivaten und Gemischen davon ausgewählt sind.

6. Kombinationspräparat nach einem der Ansprüche 1 bis 5, **dadurch gekennzeichnet, daß** es als Antikoagulans Fucoidan enthält, vorzugsweise niedermolekulares Fucoidan, besonders bevorzugt in einer Menge von 0,1-10 Gew.-%.

7. Kombinationspräparat nach einem der Ansprüche 1 bis 6, **dadurch gekennzeichnet, daß** es als Vasoprotektivum Aescin enthält, vorzugsweise in einer Menge von 0,1-7 Gew.-%.

8. Kombinationspräparat nach einem der Ansprüche 1 bis 7, **dadurch gekennzeichnet, daß** es als mikrozirkulationsfördernden Stoff Koffein enthält, vorzugsweise in einer Menge von 0,1 - 2 Gew.-%.

9. Kombinationspräparat nach einem der Ansprüche 1 bis 8, **dadurch gekennzeichnet, daß** es Aescin, vorzugsweise in einer Menge von 4,0 bis 6,0 Gew.-%, besonders bevorzugt 5,0 Gew.-%, niedermolekulares Fucoidan, vorzugsweise in einer Menge von 1,0 bis 3,0 Gew.-%, besonders bevorzugt 2,0 Gew.%, und Koffein, vorzugsweise in einer Menge von 0,5 bis 1,5 Gew.%, besonders bevorzugt 1,0 Gew.% enthält.

10. Kombinationspräparat nach einem der Ansprüche 1 bis 9, **dadurch gekennzeichnet, daß** das Trägersystem weiterhin Linolsäure in stabilisierter Form, vorzugsweise in einer Menge von 2,5-4,5 Gew.-% enthält.

11. Kombinationspräparat nach einem der Ansprüche 1 bis 10, **dadurch gekennzeichnet, daß** es weiterhin wenigstens einen Thermorezeptor-Agonisten enthält, der aus der Gruppe ausgewählt ist, die natürliches oder synthetisches Capsalcin, bevorzugt in einer Menge von 0,1 bis 1 Gew.-%, besonders bevorzugt in einer Menge von 0,2 bis 0,6 Gew.-%, und Nikotinsäure. Nicotinsäureamid, Nicotinsäureester oder Gemische davon, bevorzugt in einer Menge von 0,5 bis 5 Gew.-%, besonders bevorzugt in einer Menge von 0,5 bis 3 Gew.-%, umfaßt.

12. Kombinationspräparat nach einem der Ansprüche 1 bis 11, **dadurch gekennzeichnet, daß** es weiterhin 10-25 Gew.-% Ethanol enthält.

13. Verwendung von membranbildenden Lipiden und Wirkstoffen zur Herstellung eines Kosmetikums oder Arzneimittels für die Prophylaxe und/oder die Behandlung von Hämatomen, vorzugsweise von Hämatomen des unteren Augenlids, und/oder von Venenleiden, **dadurch gekennzeichnet, daß** eine Dreifachkombination von Wirkstoffen vorliegt, die Wirkstoffe aus den Gruppen Antikoagulantien und mikrozirkulationsfördernde Stoffe und Vasoprotektiva enthält, wobei der wenigstens eine mikrozirkulationsfördernde Stoff unter Koffein, Naftidrofuryl, Pentoxifyllin, Buflomedil, Ginkgowirkstoffen und Gemischen davon ausgewählt ist und das wenigstens eine Vasoprotektivum unter Aescin, Rutin, Diosmin, Ruscogenin und Gemischen davon ausgewählt ist.

## Claims

1. Combined cosmetic or therapeutic preparation having a carrier system comprising membrane-forming lipids and active ingredients, **characterized in that** there is a triple combination of active ingredients comprising active ingredients from the groups anti-coagulants and microcirculation-promoting substances and vasoprotective agents, wherein the at least one microcirculation-promoting substance is selected from caffeine, naftidrofuryl, pentoxyfyllin, buflomedil, ginkgo active ingredients and mixtures thereof, and wherein the at least one vasoprotective agent is selected from aescin, rutin, diosmin, ruscogenin and mixtures thereof.

2. A combined preparation according to claim 1 **characterised in that** the carrier system is vesicular.

3. A combined preparation according to claim 1 or 2 **characterised in that** the membrane-forming lipids include the groups of phospholipids, ceramides and diacylglycosides and mixtures thereof.

4. A combined preparation according to one of claims 1 to 3 **characterised in that** the membrane-forming lipids contain at least 70 % by weight of phosphatidylcholine, preferably about 80-90 % by weight of phosphatidylcholine.

5. A combined preparation according to one of claims 1 to 4 **characterised in that** the anti-coagulants are selected from heparins, fucoidans, hirudins, pentapeptides, coumarin derivatives and mixtures thereof.

6. A combined preparation according to one of claims 1 to 5 **characterised in that** as the anti-coagulant it contains fucoidan, preferably low-molecular fucoidan, particularly preferably in an amount of 0.1-10 % by weight.

7. A combined preparation according to one of claims 1 to 6 **characterised in that** it contains aescin as the vasoprotective agent, preferably in an amount of 0.1-7 % by weight.

8. A combined preparation according to one of claims 1 to 7 **characterised in that** it contains caffeine as the microcirculation-promoting substance, preferably in an amount of 0.1 - 2 % by weight.

9. A combined preparation according to one of claims 1 to 8 **characterised in that** it contains aescin, preferably in an amount of 4.0 to 6.0 % by weight, particularly preferably 5.0 % by weight, low-molecular fucoidan, preferably in an amount of 1.0 to 3.0 % by weight, particularly preferably 2.0 % by weight, and caffeine, preferably in an amount of 0.5 to 1.5 % by weight, particularly preferably 1.0 % by weight.

10. A combined preparation according to one of claims 1 to 9 **characterised in that** the carrier system additionally contains linoleic acid in stabilised form, preferably in an amount of 2.5 to 4.5 % by weight.

11. A combined preparation according to one of claims 1 to 10 **characterised in that** further contains at least one thermoreceptor-agonist which is selected from the group which includes natural or synthetic capsaicin, preferably in an amount of 0.1 to 1 % by weight, particularly preferably in an amount of 0.2 to 0.6 % by weight, and nicotinic acid, nicotinic acid amide, nicotinic acid ester or mixtures thereof, preferably in an amount of 0.5 to 5 % by weight, particularly preferably in an amount of 0.5 to 3 % by weight.

12. A combined preparation according to one of claims 1 to 11 **characterised in that** it further contains 10 - 25 % by weight of ethanol.

13. Use of membrane-forming lipids and active ingredients for the production of a cosmetic or drug for prophylaxis and/or treatment of haematomas, preferably haematomas of the lower eyelid, and/or vein complaints **characterized in that** there is a triple combination of active ingredients comprising active ingredients from the groups anti-coagulants and microcirculation-promoting substances and vasoprotective agents, wherein the at least one microcirculation-promoting substance is selected from caffeine, naftidrofuryl, pentoxyfyllin, buflomedil, ginkgo active ingredients and mixtures thereof, and wherein the at least one vasoprotective agent is selected from aescin, rutin, diosmin, ruscogenin and mixtures thereof.

## Revendications

1. Préparation combinée cosmétique ou thérapeutique comprenant un système support composé de lipides formant une membrane et de substances actives, **caractérisée par** la présence d'une triple combinaison de substances actives contenant des substances actives composées des groupes anticoagulants et des substances stimulant la microcirculation et des vasoprotecteurs, la ou les substances stimulant la microcirculation étant choisies dans le groupe constitué par la caféine, le naftidrofuryle, la pentoxifylline, le buflomédil, les substances actives à base de ginkgo et leurs mélanges et le ou les vasoprotecteurs étant choisis dans le groupe constitué par l'aescine, la rutine, la diosmine, la ruscogénine et leurs mélanges.

2. Préparation combinée selon la revendication 1, **caractérisée en ce que** le système support est vésiculaire.

3. Préparation combinée selon l'une quelconque des revendications 1 ou 2, **caractérisée en ce que** les lipides formant une membrane comportent les groupes des phospholipides, des céramides et des diacylglucosides et leurs mélanges.

4. Préparation combinée selon l'une quelconque des revendications 1 à 3, **caractérisée en ce que** les lipides formant une membrane comportent au moins 70% en poids de phosphatidylcholine, de préférence environ 80 - 90% en poids de phosphatidylcholine.

5. Préparation combinée selon l'une quelconque des revendications 1 à 4, **caractérisée en ce que** les anticoagulants sont choisis dans le groupe constitué par les héparines, les fucoïdanes, les hirudines, les pentapeptides, les dérivés coumariniques et leurs mélanges.

6. Préparation combinée selon l'une quelconque des revendications 1 à 5, **caractérisée en ce qu'**elle contient comme anticoagulant du fucoïdane, de préférence du fucoïdane à faible poids moléculaire, idéalement dans une quantité allant de 0,1 à 10% en poids.

7. Préparation combinée selon l'une quelconque des revendications 1 à 6, **caractérisée en ce qu'**elle contient comme vasoprotecteur de l'aescine, de préférence dans une quantité allant de 0,1 à 7% en poids.

8. Préparation combinée selon l'une quelconque des revendications 1 à 7, **caractérisée en ce qu'**elle contient comme substance stimulant la microcirculation de la caféine, de préférence dans une quantité allant de 0,1 à 2% en poids.

9. Préparation combinée selon l'une quelconque des revendications 1 à 8, **caractérisée en ce qu'**elle contient de l'aescine, de préférence dans une quantité allant de 4,0 à 6,0% en poids, idéalement de 5,0% en poids, du fucoïdane à faible poids moléculaire, de préférence dans une quantité allant de 1,0 à 3,0% un poids, idéalement de 2,0% en poids et de la caféine, de préférence dans une quantité allant de 0,5 à 1,5 % en poids, idéalement de 1,0% en poids.

10. Préparation combinée selon l'une quelconque des revendications 1 à 9, **caractérisée en ce que** le système support contient en outre de l'acide linoléique sous forme stabilisée, de préférence dans une quantité allant de 2,5 à 4,5% en poids.

11. Préparation combinée selon l'une quelconque des revendications 1 à 10, **caractérisée en ce qu'**elle contient en outre au moins un récepteur agoniste choisi dans le groupe constitué par la capsaïcine naturelle ou synthétique, de préférence dans une quantité allant de 0,1 à 1% en poids, idéalement dans une quantité allant de 0,2 à 0,6% en poids et de l'acide nicotinique, de la nicotisamide, l'ester de l'acide nicotinique ou leurs mélanges, de préférence dans une quantité allant de 0,5 à 5% en poids, idéalement dans une quantité allant de 0,5 à 3% en poids.

12. Préparation combinée selon l'une quelconque des revendications 1 à 11, **caractérisée en ce qu'**elle contient en outre 10 à 25% en poids d'éthanol.

13. Utilisation de lipides formant une membrane et de substances actives destinées à fabriquer un cosmétique ou un médicament pour la prophylaxie et/ou le traitement d'hématomes, de préférence d'hématomes de la paupière inférieure, et/ou d'affections veineuses, **caractérisée par** la présence d'une triple combinaison de substances actives contenant des substances actives composées des groupes anticoagulants et substances stimulant la microcirculation et des vasoprotecteurs, la ou les substances stimulant la microcirculation étant choisies dans le groupe constitué par la caféine, le naftidrofuryle, la pentoxifylline, le buflomédil, les substances actives à base de ginkgo et leurs mélanges et le ou les vasoprotecteurs étant choisis dans le groupe constitué par l'aescine, la rutine, la diosmine, la ruscogénine et leurs mélanges.
